(19) Europäisches Patentamt
European Patent Office
Office européen des brevets

(11) Veröffentlichungsnummer: **0 061 785**
**B2**

(12) **NEUE EUROPÄISCHE PATENTSCHRIFT**

(45) Veröffentlichungstag der neuen Patentschrift: **16.03.88**

(51) Int. Cl.⁴: **C 07 D 211/58,** C 08 K 5/34, C 08 G 59/50

(21) Anmeldenummer: **82104017.7**

(22) Anmeldetag: **09.01.80**

(54) **Verwendung von Polyalkylpiperid-polyaminen als Epoxydharzhärter.**

(30) Priorität: **15.01.79 CH 357/79**

(43) Veröffentlichungstag der Anmeldung:
**06.10.82 Patentblatt 82/40**

(45) Bekanntmachung des Hinweises auf die Patenterteilung:
**20.02.85 Patentblatt 85/8**

(45) Bekanntmachung des Hinweises auf die Entscheidung über den Einspruch:
**16.03.88 Patentblatt 88/11**

(84) Benannte Vertragsstaaten:
**BE CH DE FR GB IT**

(56) Entgegenhaltungen:
**WO-A-81/01706**
**CH-A-592 129**
**US-A-4 104 248**

(73) Patentinhaber: **CIBA- GEIGY AG, Klybeckstrasse 141, CH- 4002 Basel (CH)**

(72) Erfinder: **Nikles, Erwin, Dr., Bodenackerstrasse 14, CH- 4410 Liestal (CH)**

**EP 0 061 785 B2**

LIBER, STOCKHOLM 1988

**Beschreibung**

In der Stammanmeldung, jetzt EP-B-13665, werden aliphatische Polyamine, die durch eine Polyalkylpiperidingruppe substituiert sind, als Zwischenprodukte zur Herstellung von Polyamino-1,3,5-triazinen verwendet. Sowohl die Endprodukte wie die Zwischenprodukte sind Lichtschutzmittel. In der DE-A-2 805 821 sind Komplexe bestimmter Metallsalze mit solchen Polyalkylpiperidin-polyaminen als Lichtschutzmittel beschrieben, z. B. auf Seite 28 und 29 dieser Offenlegungsschrift. Ähnliche Polyamine wurden auch bereits in der DE-OS 2 349 962 und der US-A-4 104 248 als Lichtschutzmittel beschrieben.

Gegenstand der vorliegenden Erfindung ist die Verwendung solcher Polyalkylpiperidin-polyamine als Härter für Epoxydharze. Einfache aliphatische Polyamine, wie z. B. Diäthylentriamin oder Triäthylentetramin sind seit langem als Härter für Epoxydharze bekannt, siehe z. B. H. Jahn, Epoxidharze, VEB Verlag f. Grundstoffindustrie, Leipzig 1969, Seite 46. Gegenüber diesen bekannten Polyamin-Härtern zeichnen sich die erfindungsgemässen Härter dadurch aus, dass sie eine Stabilisierung des gehärteten Epoxydharzes gegen Abbau durch Witterungseinflüsse wie z. B. gegen Verfärbung oder Glanzverlust bewirken.

Die Erfindung betrifft die Verwendung von Polyaminen der Formel I,

$$R-NH-X-(-A\ X-)_c-NH-R$$

worin X $C_2$-$C_6$ Alkylen ist, A -O-, -S- oder -NR- ist, die Reste R unabhängig voneinander H, $C_1$-$C_{23}$ Alkyl, das durch -O- unterbrochen sein kann, $C_3$-$C_{12}$ Cycloalkyl, Aralkyl sowie Aryl gemäß untenstehenden Definitionen oder einen Rest der Formel II bedeuten

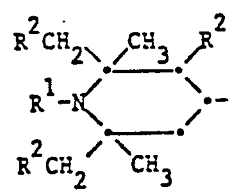

(II)

worin $R^1$ H, $C_1$-$C_{18}$ Alkyl, $C_3$-$C_{12}$ Alkenyl, $C_3$-$C_{18}$ Alkoxylalkyl, gegebenenfalls durch Phenyl oder Phenoxy substituiertes $C_2$-$C_4$ Hydroxyalkyl oder Aralkyl gemäß untenstehender Definition ist und $R^2$ Wasserstoff oder Methyl ist, c die Zahlen 1 bis 4 bedeutet und worin mindestens en Rest R eine Gruppe der Formel II ist,

$R^1$ als $C_1$-$C_{18}$ Alkyl kann beispeilsweise Methyl, Aethyl, n-Propyl, Isopropyl, n-Butyl, sec.-Butyl, n-Pentyl, n-Hexyl, n-Octyl, 1,1,3,3-Tetramethylbutyl, n-Nonyl, Decyl, Dodecyl, Tetradecul, Hexadecyl oder Octadecyl sein. Bevorzugt sind Alkylgruppen mit 1 bis 8 Kohlenstoffatomen und insbesondere solche mit 1 bis 4 Kohlenstoffatomen, als Härter für Eopxydharze.

R als $C_1$-$C_{23}$ Alkyl kann die gleiche Bedeutung wie die oben genannten Substituenten haben und dazu noch beispielsweise Nonadecyl, Eicoxyl, Heneicosyl, Docosyl und Tricosyl, wobei es sich immer um geradkettige oder verzweigte Kohlenwasser-stoffe handeln kann.

R als $C_3$-$C_{12}$ Cycloalkyl kann beispielsweise Cyclopropyl, Cyclopentyl, Cyclohexyl, Cycloheptyl, Cyclooctyl, Cyclononyl oder Cyclododecyl sein. Bevorzugt ist Cyclohexyl.

R und $R^1$ als Aralkyl stellen 1-Phentyläthyl, 1,1-Dimethylbenzyl, 2-Phenyläthyl oder vor allem Benzyl dar.

R als Aryl bedeutet gegebenenfalls durch Methyl, Aethyl, n-Propyl, Isopropyl, n-Butyl, sec.-Butyl oder tert.-Butyl substituiertes Phenyl. Bevorzugt ist unsubstituiertes Phenyl.

$R^1$ als $C_3$-$C_{18}$ Alkenyl kann insbesondere $C_3$-$C_{12}$ Alkenyl darstellen, wie beispielsweise Allyl, Methallyl, 2-Butenyl, 2-Hexenyl, 3-Hexenyl, 2-Octenyl, 2-Dodecenyl dar. Bevorzugt ist Allyl.

$R^1$ als $C_3$-$C_5$ Alkinyl kann z. B. Propargyl 1-Butinyl, 2-Butinyl oder n-1-Pentinyl sein. Bevorzugt ist Propargyl.

Stellt $R^1$ $C_3$-$C_{18}$ Alkoxyalkyl dar, so kann der Alkylteil 1 bis 3 Kohlenstoffatome enthalten und der Alkoxyteil aus 1 bis 16 Kohlenstoffatomen bestehen, wie z. B. Äthoxymethyl, 2-Methoxyäthyl, 2-Aethoxyäthyl, 2-n-Butoxyäthyl, 3-n-Butoxyäthyl, 2-Octoxyäthyl oder 2-Hexadecyloxyäthyl. Bevorzugt sind Alkoxyalkylgruppen mit 3 oder 4 Kohlenstoffatomen, wie z. B. Aethoxymethyl, 2-Methoxyäthyl oder 2-Aethoxyäthyl.

Bedeutet $R^1$ $C_2$-$C_4$ Hydroxyalkyl, so kann dies beispielsweise 2-Hydroxy-n-butyl, 2-Hydroxy-n-propyl und insbesondere 2-Hydroxyäthyl sein.

X stellt als $C_2$-$C_6$ Alkylen z. B. Aethylen, Propylen, Tetramethylen, Pentamethylen und Hexamethyl dar. Bevorzugt sind Äthylen und Propylen.

Die Polyamine der Formel I, worin ein R ein Rest der Formel II ist und das andere R Wasserstoff oder ebenfalls ein Rest der Formel R ist, können durch katalytische Hydrierung eines Ketones der Formel III

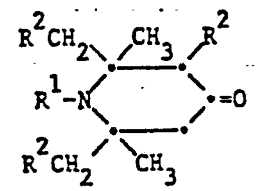

(III)

in Gegenwad eines Polyamines der Formel IV
$H_2N-X-(-A-X-)_C-NH_2$
hergestellt werden.

Die Reaktion kann unter den für reduktive Aminierungen allgemein bekannten Bedingungen durchgeführt werden. Vorzugsweise arbeitet man in Lösung, beispielsweise in Methanol. Als Katalysator verwendet man vorzugsweise Edelmetallkatalysatoren beispielsweise Platin. Je nach dem Komponentenverhältnis von III : IV erhält man ein Produkt der Formel I, in welchem nur ein Rest R ein Rest der Formel II ist und das andere R Wasserstoff ist oder in welchem beide Reste R ein Rest der Formel II sind oder ein Gemisch der beiden möglichen Produkte.

Die Ausgangsverbindungen III und IV sind allgemein bekannt und teilweise im Handel erhältlich.

Verbindungen der Formel I, worin ein R ein Rest der Formel II ist und das andere R ein Alkyl-, Cycloalkyl-, Aralkyl- oder Arylrest ist, können analog durch katalytische Hydrierung von III in Gegenwart eines Polyamines der Formel $H_2N-X-(A-X)_c-NH-R$, worin R ein Alkyl-, Cycloalkyl-, Aralkyl- oder Arylrest ist, hergestellt werden.

Verbindungen der Formel I, worin ein R ein Rest der Formel II ist und das andere R ein Alkyl-, Cycloalkyl- oder Aralkylrest lt. vorstehenden Definitionen ist, können auch durch katalytische Hydrierung einer entsprechenden aliphatischen, cycloaliphatischen oder araliphatischen Carbonylverbindung in Gegenwart eines Amines der Formel

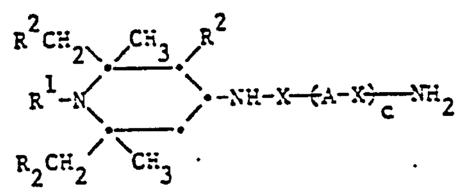

hergestellt werden.

Die Polyamine der Formel I sind als Härter für Epoxydharze verwendbar. Die sekundären und primären Aminogruppen der Polyamine reagieren dabei mit den Epoxydgruppen, wobei eine Aushädung zu vernetzten Harzen eintritt.

Die folgenden Beispiele erläutern die Herstellung der Polyamine und ihre Verwendung.

**Beispiel 1**

100 g Triacetonamin und 36,5 g Diäthylentriamin wurden in 1,4 l Methanol gelöst, mit 1,5 g konz. Schwefelsäure versetzt und in Gegenwart von 5 g 5 %-iger Platinkohle bei 20 bis 25° unter Normaldruck in einer Wasserstoffatmosphäre geschüttelt. Nach 4 Stunden war die Wasserstoffaufnahme beendet (15,4 1,0°, 760 mm Hg).

Der Katalysator wurde abfiltriert, das Lösungsmittel abgedampft und der Rückstand im Hochvakuum destilliert. Siedepunkt 161°/0,08 mmHg. Ausbeute 92 g 1,7-Bis-(2,26,-tetramethyl-4-piperidyl)-1,4,7-triazaheptan als leicht gelbliche Flüssigkeit.

**Beispiel 2**

Analog wie im Beispiel 1 beschrieben wurden 310 g Triacetonamin und 160 g Triäthylentetramin in 3 l Methanol in Gegenwart von 2,5 g konzentrierter Schwefelsäure und 15 g 5 %-ige Platinkohle hydriert. Das erhaltene 1,10-Bis(2,2,6,6-tetramethyl-4-piperidyl)-1,4,7,10-tetraazadecan siedet bei 182°/0,01 mmHg.

**Beispiel 3**

Analog wie im Beispiel 1 beschrieben, wurde die Mischung von 155 g Triacetonamin, 113 g Diäthylentriamin, 2,5 g konzentrierter Schwefelsäure in 2,5 l Methanol in Gegenwart von 7 g 5 %-iger Platinkohle hydriert. Die Destillation des Hydriergemisches ergab neben dem 1,7-Bis(2,2,6,6-tetramethyl-4-piperidyl)-1,4,7-triazaheptan bei 115 bis 118°/0,9 mmHg das 1-(2,2,6,6-Tetramethyl-4-piperidyl)-1,4,7-triaza-heptan als Hauptprodukt.

**Beispiel 4**

Analog wie im Beispiel 1 beschrieben, wurden die folgenden Polyamine hergestellt:

a) 1,9-Bis(2,2,6,6-tetramethyl-4-piperidyl)-1,5,9-triazanonan Kp. 183°C/0,666 mbar (Siedepunkt 183°/0,5 mmHg) aus Triacetonamin und Dipropylentriamin.

b) 1,9-Bis(2,2,6,6-tetramethyl-4-piperidyl)-5-methyl-1,5,9-triazanonan Kp. 200°C/0,666 mbar (Siedepunkt 200°/0,5 mmHg) aus Triacetonamin und Bis-(3-aminopropyl)-methylamin.

c) 1,17-Bis(2,2,6,6-tetramethyl-4-piperidyl)-1,5,9,13,17-pentazaheptadecan, Kp. 200°C/0,0013 mbar (Siedepunkt 200°/0,001 mmHg) aus Triacetonamin und Tetrapropylenpentamin.

d) 1,13-Bis(2,2,6,6-tetramethyl-4-piperidyl)-1,5,9,13-tetraazatridecan, Kp. 176°C/0,0065 mbar (Siedepunkt 176°/0,005 mmHg) aus Triacetonamin und Tripropylentetramin.

e) 1,14-Bis(2,2,6,6-tetramethyl-4-piperidyl)-1,14-diaza-5,10-dioxatetradecan, Kp. 173°C/0,0013 mbar (Siedepunkt 173°/0,001 mmHg) aus Triacetonamin und 4,9-Dioxatetradecan-1,12-diamin, in Gegenwart von 78 g 5 %-iger Platinkohle.

f) 1,9-Bis(2,2,6,6-tetramethyl-4-piperidyl)-5-cyclohexyl-1,5,9-triazanonan aus Triacetonamin und Bis(2-aminopropyl)-cyclohexylamin.

g) 1,9-Bis(2,2,6,6-tetramethyl-4-piperidyl)-5-n-butyl-1,5,9-triazanonan aus Triacetonamin und Bis(3-aminopropyl)butylamin.

h) 1,9-Bis(2,2,6,6-tetramethyl-4-piperidyl)-5-octadecyl-1,5,9-triazanonan aus Triacetonamin und Bis(3-aminopropyl)-octadecylamin.

i) 1,9-Bis(2,2,6,6-tetramethyl-4-piperidyl)-5-cyclododecyl-1,5,9-triazanonan aus Triacetonamin und Bis(3-aminopropyl)-cyclododecylamin.

k) 1,9-Bis(2,2,6,6-tetramethyl-4-piperidyl)-5-benzyl-1,5,9-triazanonan aus Triacetonamin und Bis(3-aminopropyl)-benzylamin.

l) 1,9-Bis(2,2,6,6-tetramethyl-4-piperidyl)-5-phenyl-1,5,9-triazanonan aus Triacetonamin und Bis(3-aminopropyl)-anilin.

m) 1,9-Bis(2,2,6,6-tetramethyl-4-piperidyl)-1,9-diaza-5-oxa-nonan, Kp. 154°C/0,065 mbar (Sdp. 154°/0,05 mmHg) aus Triacetonamin und Bis(3-aminopropyl)-äther.

n) 1,9-Bis(1,2,2,6,6-pentamethyl-4-piperidyl)-1,9-diaza-5-oxa-nonan aus N-Methyl-triacetonamin und Bis(3-aminopropyl)-äther.

o) Mischung von 1-(1,2,2,6,6-Pentamethyl-4-piperidyl)-1,4,7-triazaheptan und 1,7-Bis(1,2,2,6,6-Penta-methyl-4-piperidyl)-1,4,7-triazaheptan, Kp. 143-162°C/0,266 mbar (Sdp. 143-162°/0,2 mmHg) aus 1,2,2,6,6-Pentamethyl-4-piperidon und Diäthylentriamin.

p) 1,7-Bis(2,6-diäthyl-2,3,6-trimethyl-4-piperidyl)-1,4,7-triazaheptan, Kp. 160°C/0,0266 mbar (Sdp. 160°/0,02 mmHg, nach mehrfacher Destillation in einer Molekulardestillationsapparatur) aus 2,3,6-Trimethyl-2,6-diäthyl-4-piperidon und Diäthylentriamin in Gegenwart von 5 % (bezogen auf die Edukte) 5 %-ige Platinkohle.

**Beispiel 5**

Analog wie im Beispiel 1 beschrieben wurden die folgenden Polyamine hergestellt:

a) 1-Isobutyl-7(2,2,6,6-tetramethyl-4-piperidyl)-1,4,7-triazaheptan, Kp. 115°C/0,065 mbar (Sdp. 115°/0,05 mmHg) aus 1-(2,2,6,6-Tetramethyl-4-piperidyl)-1,4,7-triazaheptan und Isobutyraldehyd.

b) 1-Cyclohexyl-7(2,2,6,6-tetramethyl-4-piperidyl)-1,4,7-triazaheptan, Kp. 151°C/0,065 mbar (Sdp. 151°/0,05 mmHg) aus 1-(2,2,6,6-Tetramethyl-4-piperidyl)-1,4,7-triazaheptan und Cyclohexanon.

c) 1-Benzyl-7(2,2,6,6-tetramethyl-4-piperidyl)-1,4,7-triazaheptan, Kp. 172°C/0,533 mbar (Sdp. 172°/0,4 mmHg) aus 1-(2,2,6,6-Tetramethyl-4-piperidyl)-1,4,7-triazaheptan und Benzaldehyd.

d) 1-Isopropyl-7(2,2,6,6-tetramethyl-4-piperidyl)-1,4,7-triazaheptan, Kp. 115°C/0,065 mbar (Sdp. 115°/0,05 mmHg) aus 1-(2,2,6,6-Tetramethyl-4-piperidyl)-1,4,7-triazaheptan und Aceton.

**Beispiel 6**

Verwendung als Härter für Epoxydharze

a) Formkörper

Es werden Epoxydharzplatten von 135 x 135 x 4 mm hergestellt durch Mischen eines Epoxydharzes auf Basis Bisphenol A (Epoxydgehalt 5,35 Aequiv./kg) mit verschiedenen Mengen der in der Tabelle angegebenen Polyamine und Aushärtung in einer Aluminiumform im Trockenschrank 24 Stunden bei 40°C und 6 Stunden bei 100°C.

Aus den Platten werden Prüfstreifen von 60 x 10 x 4 mm geschnitten, an denen die Biegefestigkeit und Durchbiegung gemäss VSM*) -Norm 77 103 sowie die Schlagbiegefestigkeit gemäss VSM*) -Norm 77 105 bestimmt werden. Ausserdem wird die Wasseraufnahme bei Kaltwasser- und bei Kochwasserlagerung bestimmt.

Aus den Platten werden weiterhin Prüfstreifen 120 x 10 x 4 mm geschnitten, an denen die Wärmeformbeständigkeit nach ISO-Norm 75 (Heat Distortion) bestimmt wird.

Schliesslich wird noch die Glasumwandlungstemperatur ($T_g$) durch Differential-Thermoanalyse (DTA) ermittelt. Die Resultate sind in Tabelle 1 aufgeführt.

Tabelle 1

| Verwendetes Polyamin | Produkt aus Beispiel 1 | | Produkt aus Beispiel 2 | |
|---|---|---|---|---|
| Menge Polyamin in Teilen pro 100 Teilen Epoxydharz | 67,8 | 40,7 | 62,2 | 41,2 |
| Beigefestigkeit ($N/mm^2$) | 145/120 | 139 | 137/108 | 140 |
| Durchbiegung (mm) | 7,0/10,9 | 6,0 | 7,0/12,5 | 7,5 |
| Schlagbiegefestigkeit ($KJ/m^2$) | 20,5 | 15,5 | 27,0 | 21,0 |
| $H_2O$-Aufnahme, kalt (%) | 0,45 | 0,35 | 0,40 | 0,33 |
| $H_2$-Aufnahme, heiss (%) | 0,55 | 0,38 | 0,56 | 0,42 |
| $T_G$ (DTA) (°C) | 109 | 118 | 102 | 112 |
| Heat Distortion (°C) | 101 | 99 | 94 | 99 |

b) Beschichtungen

Das oben verwendete Epoxydharz wird mit 50 Teilen Titandioxidpigment (Rutil) pro 100 Teilen Epoxydharz auf einem Dreiwalzenmischwerk vermischt. Dazu werden die beiden unter a) verwendeten Polyamine in zwei verschidenen Mengenverhältnissen zugemischt. Die so erhaltenen Beschichtungsmassen werden in einer Stärke von 200 µm auf Eisenbleche aufgetragen und 24 Stunden bei 40°C und 6 Stunden bei 100°C ausgehärtet und anschliessend in einem Atlas Weatherometer 6 500 W 500 Stunden belichtet und bewittert. Vor und nach der Belichtung wurden der Weisston der Beschichtung (photometrisch) und der Glanz (bei 60°) gemessen. Die Resultate sind in Tabelle 2 aufgeführt. Aus der Differenz der Werte vor und nach der Beschichtung ergibt sich die stabilisierende Wirkung der Polyamine. Als Vergleich wurden Proben derselben Epoxydharzes mit Diäthylentriamin und Triäthylendiamin unter denselben Bedingungen gehärtet und belichtet.

| Verwendetes Polyamin (Teile auf 100 Teile Epoxydharz) | Weisston vor | nach Belichtung | Glanz vor | nach Belichtung |
|---|---|---|---|---|
| Produkt aus Beispiel 1 | | | | |
| 67,8 Teile | 2 | 3,5 | 93 | 96 |
| 40,7 Teile | 1 | 2 | 93 | 93 |
| Produkt aus Beispiel 2 | | | | |
| 62,2 Teile | 1 | 8 | 93 | 80 |
| 41,5 Teile | 1 | 7,5 | 98 | 88 |
| Diäthylentriamin | | | | |
| 11 Teile | 2 | 19 | 81 | 68 |
| Triäthylentetramin | | | | |
| 13 Teile | 2 | 16 | 54 | 56 |

*)VSM - Verband Schweizer Maschinenindustrieller

**Patentansprüche**

1. Verwendung von Polyaminen der Formel I.

R-NH-X-(-A-X-)$_c$-NH-R

worin X $C_2$-$C_6$ Alkylen ist, A-O-, -S- oder -NR- ist, die Reste R unabhängig voneinander H. $C_1$-$C_{23}$ Alkyl, das durch -O- unterbrochen sein kann. $C_3$-$C_{12}$ Cycloalkyl, Aralkyl in Form von 1-Phentyläthyl, 1,1-Dimethylbenzyl, 2-Phenyläthyl oder Benzyl, Aryl in Form von gegebenfalls durch Methyl, Ethyl, n-Propyl, Isopropyl, n-Butyl, sec.-Butyl der tert.-Butyl substituierten Phenyl oder einen Rest der Formel II bedeuten

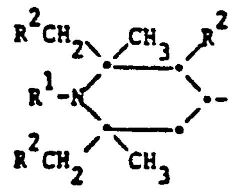

(II)

worin $R^1$ H, $C_1$-$C_{18}$ Alkyl, $C_3$-$C_{12}$ Alkenyl, $C_3$-$C_5$ Alkinyl, $C_3$-$C_{18}$ Alkoxylalkyl, gegebenenfalls durch Phenyl oder Phenoxy substituiertes $C_2$-$C_4$ Hydroxyalkyl oder Aralkyl gemäß obenstehender Definition für R ist und $R^2$ Wasserstoff oder Methyl ist, c die Zahlen 1 bis 4 bedeutet und worin mindestens ein Rest R eine Gruppe der Formel II ist, als Härter für Epoxydharze.

**Claim**

1. Use of a polyamine of the formula I
R-NH-X-(-A-X-)$_c$-NH-R (I)
wherein
X is $C_2$-$C_6$ alkylene, A is -O-, -S- or -NR-, each of the radicals R independently of the other is hydrogen, $C_1$-$C_{23}$ alkyl which may be interrupted by -O-, or is $C_3$-$C_{12}$ cycloalkyl, or aralkyl in the form of 1-phenylethyl, 1,1-dimethylbenzyl, 2-phenylethyl or benzyl, or is aryl in the form of phenyl which is unsubstituted or substituted by methyl, ethyl, n-proyl, isopropyl, n-butyl, sec-butyl or tert-butyl, or is a radical of the formula II

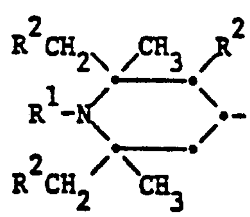

(II)

wherein
$R^1$ is hydrogen, $C_1$-$C_{18}$ alkyl, $C_3$-$C_{12}$ alkenyl, $C_3$-$C_5$ alkynyl, $C_3$-$C_{18}$ alkoxyalkyl, $C_2$-$C_4$ hydroxyalkyl which is unsubstituted or substituted by phenyl or phenoxy, or is aralkyl as defined above for R, and $R^2$ is hydrogen or methyl, c is 1 to 4, and wherein at least one of the radicals R is a group of the formula II, as curing agent for epoxy resins.

**Revendication**

1. Application des polyamines répondant à la formule I:
R-NH-X-(-A-X-)$_c$-NH-R (I)
dans laquelle
X représente un alkylène en $C_2$-$C_6$,
A représente -O-, -S- ou -NR-,
les R représentent chacun, indépendamment les uns des autres, un atome d'hydrogène, un alkyle en $C_1$-$C_{23}$ éventuellement interrompu par -O-, un cycloalkyle en $C_3$-$C_{12}$ , un aralkyle sous la forme d'un radical phényl-1 éthyle, phényl-1 méthyl-1 éthyle, phényl-2 éthyle ou benzyle, un aryle sous la forme d'un phényle éventuellement porteur d'un radical méthyle, éthyle, n-propyle, isopropyle, n-butyle, sec-butyle ou tert-butyle, ou un radical répondant à la formule II:

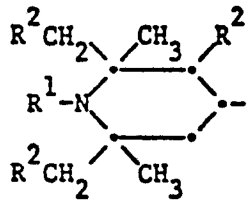 (II) ,

dans laquelle $R_1$ représente H, un alkyle en $C_1$-$C_{18}$, un alcényle en $C_3$-$C_{12}$, un alcynyle en $C_3$-$C_5$, un alcoxyalkyle en $C_3$-$C_{18}$, un hydroxy-alkyle en $C_2$-$C_4$, éventuellement porteur d'un phényle ou d'un phénoxy, ou un aralkyle conforme à la définition donnée ci-dessus pour R, et $R^2$ représente l'hydrogène ou un méthyle,

et

c représente un nombre de 1 à 4, et dans laquelle au moins l'un des symboles R est un radical de formule II, comme durcisseurs de résines époxydiques.